(19) Europäisches Patentamt
European Patent Office
Office européen des brevets

(11) Numéro de publication: **0 519 808 B1**

(12) # FASCICULE DE BREVET EUROPEEN

(45) Date de publication de fascicule du brevet: **28.12.94**

(51) Int. Cl.$^5$: **C07C 19/08**, C07C 17/20

(21) Numéro de dépôt: **92401674.4**

(22) Date de dépôt: **16.06.92**

(54) **Synthèse d'un bromure de perfluoroalkyle par photobromation de l'iodure correspondant.**

(30) Priorité: **21.06.91 FR 9107649**

(43) Date de publication de la demande:
**23.12.92 Bulletin 92/52**

(45) Mention de la délivrance du brevet:
**28.12.94 Bulletin 94/52**

(84) Etats contractants désignés:
**AT DK SE**

(56) Documents cités:

**CHEMICAL ABSTRACTS, vol. 104, no. 11, 17 Mars 1986, Columbus, Ohio, US; abstract no. 88106P, page 619 ;**

(73) Titulaire: **ELF ATOCHEM S.A.**
**4 & 8, Cours Michelet**
**La Défense 10**
**F-92800 Puteaux (FR)**

(72) Inventeur: **Braun, André**
**Chemin de l'Epi d'Or, 17**
**CH-1053 Cugy (CH)**
Inventeur: **Drivon, Gilles**
**3, Rue Joanny Courbière**
**F-69850 Saint Martin en Haut (FR)**
Inventeur: **Kervennal, Jacques**
**134, Rue E. Locard**
**F-69005 Lyon (FR)**

(74) Mandataire: **Leboulenger, Jean et al**
**Elf Atochem S.A.**
**Département Propriété Industrielle, Cedex 42 - La Défense 10**
**F-92091 Paris la Défense (FR)**

**EP 0 519 808 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne le domaine des bromures $R_FBr$, $R_F$ désignant un radical perfluoroalkyle, linéaire ou ramifié, contenant de 2 à 14 atomes de carbone. Elle a plus particulièrement pour objet la préparation du bromure de n.perfluorooctyle $C_8F_{17}Br$ (ci-après désigné par l'abréviation PFOB).

On sait que les composés $R_FBr$ et plus particulièrement le PFOB présentent des propriétés très intéressantes dans le domaine médical comme agents de contraste (radiologie) ou comme transporteurs d'oxygène (sang artificiel). Leur futur développement dans ces applications apparait être important. Aussi est-il nécessaire d'être en mesure de les produire industriellement par un procédé économique avec une très grande pureté.

Parmi les méthodes connues pour préparer les composés $R_FBr$, on peut d'abord signaler :

- l'action du brome sur un composé $R_FSF_5$ à 500 °C en présence de nickel (brevet Us 3 456 024) ;
- la photolyse en phase gazeuse d'un composé $R_FH$ par Br-Cl ou Br-F (J.L. Adcock et al, CA 100, 34092 e) ou par $Br_2$ (brevet FR 1 512 068).

La faiblesse des rendements obtenus et/ou l'utilisation de dérivés fluorés non disponibles industriellement ne permettent pas une production économique des composés $R_FBr$ à l'échelle industrielle.

Dans son brevet EP 0 298 870 et sa demande de brevet EP 90403118.4, la Demanderesse a décrit des procédés de fabrication des composés $R_FBr$ à partir des chlorures de perfluoroalcane-sulfonyle $R_FSO_2Cl$ correspondants qu'on fait réagir soit avec HBr gazeux en présence d'un catalyseur (EP 0 298 870), soit avec un bromure d'ammonium ou de phosphonium quaternaire (EP 90403118.4). Les rendements obtenus sont généralement élevés, mais le sulfochlorure $R_FSO_2Cl$ utilisé est une matière première déjà très élaborée puisque sa synthèse à partir de l'iodure correspondant $R_FI$ nécessite deux étapes réactionnelles suivant l'équation :

$$2\ R_FI + 2\ SO_2 \xrightarrow{Zn} (R_FSO_2)_2Zn \xrightarrow{2Cl_2} 2\ R_FSO_2Cl + ZnCl_2$$

La voie la plus directe d'obtention des composés $R_FBr$ consiste évidemment en une bromation radicalaire des iodures correspondants $R_FI$, ces derniers étant des produits disponibles en quantités industrielles.

Dans International Journal of Chemical Kinetics, Vol. II, 273-285 (1975), E.N. Okafo et E. Whittle décrivent la cinétique de bromation thermique de $CF_3I$ dans un réacteur photochimique entre 173 et 321 °C, en vue de déterminer l'énergie de dissociation de la liaison C-I.

Dans J. Chem. Soc. 1953, 3761-8, R.N. Haszeldine a décrit un procédé photochimique de réaction des $R_FI$ avec du brome en opérant en tube scellé avec un excès de brome (10 %) et en irradiant par une lumière UV pendant sept jours. La température réactionnelle et la pureté obtenue ne sont pas précisées ; il est simplement indiqué que le rendement est supérieur ou égal à 90 % selon la longueur de la chaîne perfluorée $R_F$.

Dans les exemples de la demande japonaise Kokai 85-184033 qui décrit la réaction des $R_FI$ avec $Br_2$ en présence d'un initiateur chimique de radicaux, les rendements indiqués ne dépassent pas 42 %.

La faiblesse des rendements obtenus et/ou la cinétique lente de ces techniques, ainsi que la transformation toujours incomplète du $R_FI$, ne permettent pas d'envisager une exploitation industrielle. En effet, compte tenu des applications visées (domaine médical), les composés $R_FBr$ et plus spécifiquement le PFOB doivent présenter une grande pureté, en particulier un très faible teneur en iodures $R_FI$ qui sont instables dans le temps avec développement d'une coloration rosée et difficilement séparables par distillation, leur point d'ébullition étant proche de celui du bromure correspondant.

Il a par ailleurs été constaté que, si l'on procède à la photobromation d'un $R_FI$ selon la technique classique, c'est-à-dire en introduisant le $R_FI$ et un excès de $Br_2$ dans un réacteur photochimique muni d'une lampe émettant dans les longueurs d'onde comprises entre 300 et 650 nm, le $R_FI$ se transforme en $R_FBr$ avec une cinétique assez rapide au début, puis la vitesse de la réaction diminue très rapidement pour s'arrêter à un taux de transformation global inférieur à 20 %.

Il a maintenant été trouvé qu'il est néanmoins possible de bromer photochimiquement un iodure de perfluoroalkyle $R_FI$en $R_FBr$ avec une conversion quasi-quantitative si la photobromation est effectuée en milieu dilué en utilisant comme solvant du $R_FI$, soit le composé $R_FBr$ lui-même, soit un solvant inerte tel que, par exemple, $CCl_4$ ou $C_2F_3Cl_3$.

L'invention a donc pour objet un procédé de fabrication d'un bromure de perfluoroalkyle $R_FBr$ par bromation photochimique de l'iodure correspondant $R_FI$, caractérisé en ce que l'on soumet à la photobromation une solution du $R_FI$ dans un solvant, la concentration en $R_FI$ de ladite solution étant au plus égale à 0,5 mole/litre.

La solubilité du $R_FI$ dans le solvant (de préférence $R_FBr$) est bien supérieure à 0,5 mole/litre, mais les expériences effectuées ont montré que sa concentration dans la solution soumise à la photobromation ne doit pas excéder cette valeur pour obtenir une conversion pratiquement complète de l'iodure $R_FI$. Pour la cinétique de la réaction, il est d'ailleurs préférable d'opérer à une concentration en $R_FI$ inférieure à 0,35 mole/litre et, avantageusement, inférieure à 0,2 mole/litre.

La solution de départ peut être préparée par simple mélange du $R_FI$ et du solvant, mais on peut également utiliser le produit obtenu par bromation thermique ménagée de l'iodure $R_FI$, ce produit pouvant éventuellement être dilué par addition de $R_FBr$ pour l'amener à la concentration désirée en $R_FI$.

La photobromation peut être effectuée en discontinu ou en continu, dans tout réacteur photochimique, par exemple dans un photoréacteur à immersion ou à film tombant, équipé d'une enveloppe de lampe en quartz ou pyrex et muni d'une lampe émettant dans les longueurs d'onde comprises entre 300 et 650 nm.

Le brome est de préférence introduit progressivement de façon à absorber complètement la lumière. La quantité totale de brome à utiliser est d'au moins une mole par mole de $R_FI$présent dans la solution initiale, mais on préfère utiliser un excès de brome de 10 à 100 %, en particulier 25 % environ.

La photobromation selon l'invention peut être menée à une température comprise entre 10 °C et le point d'ébullition du solvant, mais on opère de préférence entre 15 et 50 °C. L'opération est avantageusement réalisée à pression atmosphérique, mais on ne sortirait pas du cadre de la présente invention en travaillant sous légère pression ou dépression.

Le procédé selon l'invention s'applique aussi bien à la préparation d'un $R_FBr$ spécifique (par exemple $C_6F_{13}Br$, $C_8F_{17}Br$, $C_{10}F_{21}Br$, ...) qu'à celle d'un mélange de différents $R_FBr$ à partir d'un mélange des $R_FI$ correspondants.

Les exemples suivant illustrent l'invention sans la limiter. Pour suivre le déroulement de la réaction au cours du temps, on prélève des échantillons et, après réduction et élimination de l'iode et du brome libres, on les analyse par chromatographie en phase gazeuse (détecteur à conductivité thermique). Le taux de transformation global ($TT_G$) du $R_FI$ est calculé par la formule suivante :

$$TT_G = 100 \times \frac{R_FI \text{ initial} - R_FI \text{ résiduel}}{R_FI \text{ initial}}$$

La limite de détection de l'appareil étant d'environ 1000 ppm pour le $R_FI$ , on considère avoir obtenu un $TT_G$ de 100 % lorsque cette limite de détection est atteinte.

## **EXEMPLE 1** (Comparatif)

Dans un réacteur photochimique à immersion d'une capacité de 150 ml, muni d'une lampe PHILIPS HPK 125 et d'une agitation magnétique, on charge 270 g (140 ml) d'iodure de perfluorooctyle (pureté > 99,5 %), puis on irradie ce composé à 30 ± 2 °C tout en introduisant du brome, cette introduction étant effectuée goutte à goutte de telle sorte que l'absorption du brome soit totale.

Après 24 heures d'irradiation, on prélève un échantillon (2 ml) et le traite avec une solution aqueuse de sulfite de sodium. Son analyse CPG montre que la teneur en PFOB ($C_8F_{17}Br$) est de 12,5 % en poids, ce qui correspond à un $TT_G$ de 13,5 %.

On poursuit l'irradiation du mélange réactionnel pendant encore 15 heures à 55 °C. L'analyse CPG du brut réactionnel indique alors une teneur en PFOB de 18,7 % en poids, soit un $TT_G$ de seulement 20,1 %.

## **EXEMPLE 2**

Dans le même appareillage qu'à l'exemple 1, on charge 140 ml d'une solution de 10 g (18,3 millimoles) d'iodure de perfluorooctyle dans le PFOB (soit une concentration molaire en $C_8F_{17}I$ de 0,13 mole/litre).

On irradie ensuite cette solution à 15-20 °C tout en introduisant du brome goutte à goutte. Après 18 heures d'irradiation, la concentration en $C_8F_{17}I$ du brut réactionnel est devenue inférieure à la limite de détection (0,1 %). le $TT_G$ est considéré comme étant égal à 100 %.

## EXEMPLES 3 A 5

On répète l'exemple 2 en faisant varier la quantité de $C_8F_{17}I$ dissous dans le PFOB, à savoir :

- 6 g pour l'exemple 3
- 25 g pour l'exemple 4
- 50 g pour l'exemple 5 (comparatif)

le volume de solution restant le même (140 ml).

Pour les exemples 3 et 4, on obtient un $TT_G$ de 100 % après respectivement 12 heures et 49 heures d'irradiation. Dans le cas de l'exemple 5, le $TT_G$ plafonne à 75 % après 50 heures d'irradiation.

Le tableau suivant résume les résultats des exemples 1 à 5 précédents.

| EXEMPLE | $C_8F_{17}I$ dans PFOB [(a)] | | Durée d'irradiation (heures) | $C_8F_{17}I$ résiduel (% poids) | TTG % |
|---|---|---|---|---|---|
| | % poids | mole/litre | | | |
| 1 comparatif | 100 | 3,54 | 39 [(b)] | 81,3 | 20,1 |
| 2 | 3,7 | 0,13 | 18 | < 0,1 | 100 |
| 3 | 2,2 | 0,08 | 12 | < 0,1 | 100 |
| 4 | 9,25 | 0,33 | 33<br>49 | 0,2<br>< 0,1 | 98<br>100 |
| 5 comparatif | 18,5 | 0,66 | 33<br>50 | 5,6<br>4,7 | 70<br>75 |

(a) Pas de PFOB à l'exemple 1
(b) 24 heures à 30 °C, puis 15 heures à 55 °C

## EXEMPLE 6

On opère comme à l'exemple 2, mais en utilisant comme solution initiale un produit obtenu préalablement par bromation thermique de $C_8F_{17}I$. Ce produit contient 12,45 % en poids de $C_8F_{17}I$ résiduel, ce qui correspond à une concentration molaire de 0,44 mole/litre.

Après 23 heures d'irradiation à 15-20 °C, la teneur en $C_8F_{17}I$ n'est plus que de 6 %. Elle est inférieure à 0,1 % après 60 heures d'irradiation ($TT_G$ = 100 %).

## EXEMPLE 7

Dans un réacteur photochimique à film tombant d'une capacité de 1600 ml, muni d'une lampe HANAU TQ 718 de 720 watts et d'une pompe externe de recirculation, on charge 1400 ml d'une solution de 250 g (0,46 mole) de $C_8F_{17}I$ dans le PFOB (soit une concentration molaire de 0,33 mole/litre).

On irradie cette solution à 15-20 °C sous circulation, tout en y introduisant du brome goutte à goutte, la quantité totale de brome correspondant à un excès de 25 % par rapport à la stoechiométrie.

Après 48 heures d'irradiation, l'analyse CPG indique une teneur résiduelle en $C_8F_{17}I$ de 5,5 % en poids. Elle n'est plus que de 0,28 % après 200 heures d'irradiation ($TT_G$ = 97 %).

Comparativement, si on opère à partir de 1400 ml de $C_8F_{17}I$ pur, l'essai doit être arrêté après 62 heures d'irradiation en raison d'une précipitation de $I_2$ et IBr qui empêchent le bon fonctionnement de la pompe de recirculation. L'analyse CPG indique alors une teneur en PFOB de 26 % en poids (soit un $TT_G$ de 27,7 %).

## EXEMPLE 8

Dans le même appareillage qu'à l'exemple 1, on charge 140 ml d'une solution de 25 g (56 millimoles) d'iodure de n.perfluorohexyle dans le bromure de n.perfluorohexyle, puis on irradie cette solution à environ 20 ° C tout en introduisant du brome goutte à goutte.

Après 48 heures d'irradiation, la teneur résiduelle en $C_6F_{13}I$ du brut réactionnel est devenue inférieure à la limite de détection de 0,1 % ($TT_G$ = 100 %).

## EXEMPLES 9 ET 10

Dans le même appareillage qu'à l'exemple 1, on charge 140 ml d'une solution contenant 15 g de $C_8F_{17}I$ dans du tétrachlorure de carbone (exemple 9) ou dans du 1,1,2-trichloro1,2,2-trifluoroéthane (exemple 10).

L'irradiation (24 heures) est effectuée à environ 20 ° C tout en introduisant du brome goutte à goutte. Le tableau suivant rassemble les résultats obtenus.

| EXEMPLE | Solvant | $C_8F_{17}I$ résidual (% poids) | TTG % |
|---|---|---|---|
| 9 | $CCl_4$ | < 0,1 | 100 |
| 10 | $C_2F_3Cl_3$ | < 0,1 | 100 |

### Revendications

1. Procédé de fabrication d'un bromure $R_FBr$ où $R_F$ désigne un radical perfluoroalkyle linéaire ou ramifié, contenant 2 à 14 atomes de carbone, par photobromation de l'iodure de perfluoroalkyle $R_FI$ correspondant, caractérisé en ce que l'on soumet à la photobromation une solution de $R_FI$ dans un solvant, la concentration en $R_FI$ de ladite solution étant au plus égale à 0,5 mole/litre.

2. Procédé selon la revendication 1, dans lequel la concentration en $R_FI$ est inférieure à 0,35 mole/litre et de préférence inférieure ou égale à 0,2 mole/litre.

3. Procédé selon la revendication 1 ou 2, dans lequel le solvant utilisé est le $R_FBr$ lui-même.

4. Procédé selon la revendication 3, dans lequel la solution initiale de $R_FI$ dans le $R_FBr$ est obtenue préalablement par bromation thermique du $R_FI$.

5. Procédé selon la revendication 1 ou 2, dans lequel le solvant utilisé est le tétrachlorure de carbone ou le 1,1,2-trichloro-1,2,2-trifluoroéthane.

6. Procédé selon l'une des revendications 1 à 5, dans lequel on utilise au total 1,1 à 2 moles de brome par mole de $R_FI$ présent dans la solution initiale.

7. Procédé selon l'une des revendications 1 à 6, dans lequel la photobromation est effectuée à pression atmosphérique et à une température comprise entre 15 et 50 ° C.

8. Application du procédé selon l'une des revendications 1 à 7 à la synthèse du bromure de n.perfluorooctyle.

9. Application du procédé selon l'une des revendications 1 à 7 à la synthèse du bromure de n.perfluorohexyle.

10. Application du procédé selon l'une des revendications 1 à 7 à la synthèse d'un mélange de bromures de perfluoroalkyle.

## Claims

1. Method of manufacture of a bromide $R_FBr$ where $R_F$ denotes a linear or branched perfluoroalkyl radical containing from 2 to 14 carbon atoms, by photobromination of the corresponding perfluoroalkyl iodide $R_FI$, characterised in that a solution of $R_FI$ in a solvent is subjected to the photobromination, the concentration of $R_FI$ in the said solution being at most equal to 0.5 mol/litre.

2. Method according to Claim 1, in which the concentration of $R_FI$ is lower than 0.35 mol/litre and preferably lower than or equal to 0.2 mol/litre.

3. Method according to Claim 1 or 2, in which the solvent used is the $R_FBr$ itself.

4. Method according to Claim 3, in which the initial solution of $R_FI$ in the $R_FBr$ is obtained beforehand by thermal bromination of the $R_FI$.

5. Method according to Claim 1 or 2, in which the solvent used is carbon tetrachloride or 1,1,2-trichloro-1,2,2-trifluoroethane.

6. Method according to one of Claims 1 to 5, in which a total of 1.1 to 2 moles of bromine per mole of $R_FI$ present in the initial solution is used.

7. Method according to one of Claims 1 to 6, in which the photobromination is carried out at atmospheric pressure and at a temperature of between 15 and 50 °C.

8. Application of the method according to one of Claims 1 to 7 to the synthesis of n-perfluorooctyl bromide.

9. Application of the method according to one of Claims 1 to 7 to the synthesis of n-perfluorohexyl bromide.

10. Application of the method according to one of Claims 1 to 7 to the synthesis of a mixture of perfluoroalkyl bromides.

## Patentansprüche

1. Verfahren zur Herstellung eines Bromids $R_FBr$, worin $R_F$ für einen linearen oder verzweigten Perfluoralkylrest mit 2 bis 14 Kohlenstoffatomen steht, durch Photobromierung des entsprechenden Perfluoralkyliodids $R_FI$, dadurch gekennzeichnet, daß eine Lösung aus $R_FI$ in einem Lösungsmittel der Photobromierung unterzogen wird, wobei die $R_FI$-Konzentration in der genannten Lösung höchstens gleich 0,5 Mol/l ist.

2. Verfahren nach Anspruch 1, bei dem die $R_FI$-Konzentration geringer als 0,35 Mol/l und vorzugsweise geringer als oder gleich 0,2 Mol/l ist.

3. Verfahren nach Anspruch 1 oder 2, bei dem das verwendete Lösungsmittel $R_FBr$ selbst ist.

4. Verfahren nach Anspruch 3, bei dem die Ausgangslösung aus $R_FI$ in $R_FBr$ zuvor durch thermische Bromierung von $R_FI$ erhalten wird.

5. Verfahren nach Anspruch 1 oder 2, bei dem das verwendete Lösungsmittel Kohlenstofftetrachlorid oder 1,1,2-Trichlor-1,2,2-trifluoräthan ist.

6. Verfahren nach einem der Ansprüche 1 bis 5, bei dem insgesamt 1,1 bis 2 Mole Brom pro in der Ausgangslösung vorhandenen Molen $R_FI$ verwendet werden.

7. Verfahren nach einem der Ansprüche 1 bis 6, bei dem die Photobromierung bei atmosphärischem Druck und bei einer Temperatur zwischen 15 und 50 °C durchgeführt wird.

8. Anwendung des Verfahrens nach einem der Ansprüche 1 bis 7 zur Synthese von n-Perfluoroktylbromid.

9. Anwendung des Verfahrens nach einem der Ansprüche 1 bis 7 zur Synthese von n-Perfluorhexylbromid.

10. Anwendung des Verfahrens nach einem der Ansprüche 1 bis 7 zur Synthese einer Mischung aus Perfluoralkylbromiden.